# FASCICULE DE BREVET EUROPEEN

(11) **EP 3 172 357 B1**
(45) Date de publication et mention de la délivrance du brevet: **02.05.2018**
(21) Numéro de dépôt: 15742243.7
(22) Date de dépôt: 24.07.2015
(51) Int. Cl.: C23C 16/44, C23C 14/56, H01J 37/32, B08B 7/00, C23F 1/12, C23G 5/00, H01L 21/67, C07C 45/00

(54) **PROCÉDÉ D'ÉLIMINATION D'UN DÉPÔT MÉTALLIQUE DISPOSÉ SUR UNE SURFACE DANS UNE ENCEINTE**
VERFAHREN ZUR ENTFERNUNG EINES AUF EINER OBERFLÄCHE IN EINER KAMMER AUFGETRAGENEN METALLÜBERZUGS
METHOD FOR REMOVING A METAL DEPOSIT PLACED ON A SURFACE IN A CHAMBER

(30) Priorité: 24.07.2014 FR 1457137
(43) Date de publication de la demande: 31.05.2017
(73) Titulaire: KOBUS, 38330 Montbonnot-Saint-Martin (FR)
(72) Inventeur: VITIELLO, Julien, F-38100 Grenoble (FR); DELCARRI, Jean-Luc, F-38330 Saint Ismier (FR); PIALLAT, Fabien, F-38330 Montbonnot-Saint-Martin (FR)
(74) Mandataire: Pontet Allano & Associes
(86) Numéro de dépôt international: PCT/EP2015/067044
(87) Numéro de publication internationale: WO 2016/012610

(56) Documents cités:
- WO-A1-2004/003256
- DE-A1-102011 056 538
- US-A1- 2001 009 154
- SEKIGUCHI A ET AL: "Reaction of copper oxide and beta -diketone for in situ cleaning of metal copper in a copper chemical vapor deposition reactor", JAPANESE JOURNAL OF APPLIED PHYSICS, JAPAN SOCIETY OF APPLIED PHYSICS, JP, vol. 39, no. 11, 1 novembre 2000 (2000-11-01), pages 6478-6486, XP002205430, ISSN: 0021-4922, DOI: 10.1143/JJAP.39.6478

## Description

### DOMAINE DE L'INVENTION

La présente invention concerne un procédé d'élimination d'un dépôt métallique disposé sur une surface dans une enceinte.

### ARRIERE PLAN DE L'INVENTION

Un procédé d'élimination d'un dépôt métallique sur une surface d'une enceinte, connu de l'état de la technique, comprend les étapes suivantes :
a) une étape d'oxydation du dépôt métallique ;
b) une étape d'injection d'espèces chimiques adaptées pour volatiliser le dépôt métallique oxydé, ladite étape b) étant mise en oeuvre pendant au moins une partie de l'étape a).

Cependant, un tel procédé d'élimination n'est pas satisfaisant.

En effet, les espèces chimiques adaptées pour volatiliser le dépôt métallique oxydé peuvent également réagir avec le dépôt métallique avant l'oxydation de ce dernier. L'étape a) s'en trouve bloquée.

Ainsi, la réaction des espèces chimiques avec le dépôt métallique parasite le procédé d'élimination, et surtout nuit à son efficacité.

C'est notamment le cas lorsque le procédé d'élimination est exécuté pour retirer un dépôt de cuivre (Cu) sur une surface dans une enceinte. L'étape a) comprend généralement l'introduction d'oxygène gazeux ou d'ozone gazeux. Les espèces chimiques adaptées pour volatiliser l'oxyde de cuivre comprennent de l'hexafluoroacetylacetone (hfacH). L'espèce chimique hfacH réagit également avec le cuivre avant qu'il ne soit oxydé lorsqu'elle est injectée dans l'enceinte. La réaction d'oxydation est alors bloquée.

L'objet de la présente invention est alors de proposer un procédé d'élimination d'un dépôt métallique permettant de limiter les réactions parasites susceptibles de bloquer ledit procédé.

Une première application de l'invention concerne le nettoyage de résidus métalliques disposés sur les parois intérieures d'une chambre de dépôt.

Une autre application de l'invention concerne la fabrication des circuits imprimés, et plus particulièrement la gravure de couches métalliques utilisées notamment pour le remplissage de vias, qui sont des trous métallisés permettant la connexion électrique entre plusieurs couches d'un circuit imprimé.

De manière classique, les vias sont remplis en excès avec un métal tel que le cuivre, de manière à assurer un remplissage satisfaisant des vias. Le métal en excès est retiré par une étape de gravure chimico-mécanique. Une couche d'arrêt est disposée entre le substrat du circuit imprimé et la couche de dépôt métallique, pour contrôler l'épaisseur de la gravure. L'étape de gravure chimico-mécanique nécessite l'utilisation de couches d'arrêt pour assurer un contrôle très précis du procédé et requiert en outre des opérations ultérieures de nettoyage de la surface gravée, ce qui est complexe et coûteux.

Un autre objet de l'invention est de proposer un procédé de fabrication de vias métallisés simplifié et moins coûteux.

### BREVE DESCRIPTION DE L'INVENTION

La présente invention vise à remédier en tout ou partie aux inconvénients précités et concerne un procédé d'élimination d'un dépôt métallique disposé sur une surface dans une enceinte, ledit procédé comprenant les étapes suivantes :
a) une étape d'oxydation du dépôt métallique ;
b) une étape d'injection d'espèces chimiques adaptées pour volatiliser le dépôt métallique oxydé, ladite étape b) étant mise en oeuvre pendant au moins une partie de l'étape a) ;
ledit procédé d'élimination étant remarquable en ce que, à l'étape b), les espèces chimiques sont injectées selon une séquence d'impulsions, et lors de l'impulsion la plus tardive de deux impulsions successives, les espèces chimiques sont injectées en quantité sous-stoechiométrique par rapport à la quantité du dépôt métallique oxydé entre lesdites deux impulsions successives de sorte que le dépôt métallique oxydé ne soit pas intégralement volatilisé. Ainsi, l'injection, selon une séquence d'impulsions, des espèces chimiques adaptées pour volatiliser le dépôt métallique oxydé permet d'éviter la réaction parasite comprenant la réaction du dépôt métallique disposé sur une surface dans une enceinte avec lesdites espèces chimiques.

Par ailleurs, l'injection, selon une séquence d'impulsions, des espèces chimiques adaptées pour volatiliser le dépôt métallique oxydé permet également de limiter la quantité desdites espèces chimiques.

Selon l'invention à l'étape b), lors de l'impulsion la plus tardive de deux impulsions successives, les espèces chimiques sont injectées en quantité sous-stoechiométrique par rapport à la quantité du dépôt métallique oxydé entre lesdites deux impulsions successives.

Ainsi, il est possible de limiter la consommation des espèces chimiques adaptées pour volatiliser le dépôt métallique oxydé, et par conséquent de limiter le coût de l'élimination du dépôt.

Selon un mode de mise en oeuvre, la séquence d'impulsions des espèces chimiques adaptées pour volatiliser le dépôt métallique oxydé est périodique.

Selon un mode de mise en oeuvre, la période de la séquence d'impulsions est inférieure à 1 seconde.

Selon un mode de mise en oeuvre, l'enceinte est maintenue à une température comprise entre 20 et 250°C, de préférence entre 20 et 150°C.

Selon un mode de mise en oeuvre, l'étape a) est exécutée par injection d'une espèce oxydante comprenant au moins une des espèces suivantes : oxygène, ozone, protoxyde d'azote.

Selon un mode de mise en oeuvre, les espèces chimiques injectées à l'étape b) comprennent de l'hexafluoroacetylacetone.

Selon un mode de mise en oeuvre, le dépôt métallique comprend au moins un des éléments suivants : Cuivre, Titane, Tantale, Ruthénium, Zinc, Zirconium, Vanadium, Argent, Or, Chrome.

Selon un mode de mise en oeuvre, la première impulsion de l'étape b) est mise en oeuvre après le début de l'étape a).

Selon un mode de mise en oeuvre, le procédé est mis en oeuvre pour le nettoyage de résidus métalliques disposés sur les parois intérieures d'une chambre de dépôt.

Selon un mode de mise en oeuvre, le procédé est mis en oeuvre pour graver un dépôt métallique déposé en excès sur une surface.

De manière facultative, préalablement aux étapes a) et b), un masque est apposé de manière localisée sur le dépôt métallique.

Selon un mode de mise en oeuvre, le procédé comprend les étapes suivantes :
- le masque est apposé sur chaque région du dépôt métallique à conserver ;
- les étapes a) et b) sont mises en oeuvre pour éliminer l'excès du dépôt métallique dans chaque région non recouverte par le masque.

Selon un autre mode de mise en oeuvre, le procédé comprend les étapes suivantes :
- le masque est apposé sur chaque région du dépôt métallique à graver ;
- des espèces chimiques adaptées pour passiver le dépôt métallique dans chaque région non recouverte par le masque sont injectées ;
- le masque est retiré ;
- les étapes a) et b) sont mises en oeuvre pour éliminer l'excès du dépôt métallique dans chaque région préalablement recouverte par le masque.

Selon un mode de mise en oeuvre, les étapes a) et b) sont répétées autant de fois que nécessaire pour éliminer l'excès du dépôt métallique.

### DESCRIPTION DES FIGURES

L'invention sera mieux comprise à la lumière de la description qui suit des modes de réalisation particuliers non limitatifs de l'invention en référence aux figures ci-jointes parmi lesquelles :
- la figure 1 est une présentation schématique d'une enceinte ;
- la figure 2(a) représente la quantité d'espèces oxydantes injectées dans l'enceinte en fonction du temps selon un mode de réalisation de l'invention ;
- la figure 2(b) représente la quantité d'espèce chimique adaptée pour volatiliser le dépôt métallique oxydé injecté dans l'enceinte en fonction du temps selon un mode de réalisation de l'invention, l'échelle de temps étant la même que celle de la figure 2(a) ;
- les figures 3(a) et 3(b) sont des coupes transversales partielles d'un circuit imprimé, le procédé de l'invention étant mis en oeuvre selon deux variantes pour graver un dépôt métallique déposé en excès sur la surface d'un substrat du circuit imprimé.

### DESCRIPTION DETAILLEE DE MODES DE REALISATION DE L'INVENTION

Pour les différents modes de mise en oeuvre, les mêmes références seront utilisées pour des éléments identiques ou assurant la même fonction, par souci de simplification de la description.

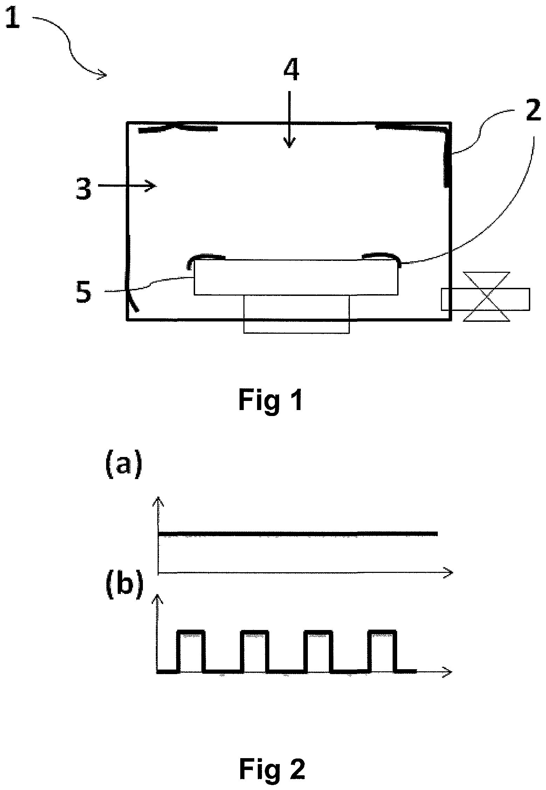

La figure 1 représente une enceinte 1 dans laquelle est disposé un support 5.

Un dépôt métallique 2 indésirable est observé sur des surfaces dans l'enceinte 1, à savoir sur les parois intérieures de l'enceinte 1, ainsi que sur le support 5.

Le dépôt métallique 2 peut comprendre au moins un des éléments suivants : Cuivre, Titane, Tantale, Ruthénium, Zinc, Zirconium, Vanadium, Argent, Or, Chrome.

Le procédé d'élimination du dépôt métallique 2 sur les surfaces dans l'enceinte 1 comprend une étape a) d'oxydation du dépôt métallique 2.

L'étape a) peut être exécutée par injection d'espèces oxydantes sous forme gazeuse par un système d'injection 3.

L'étape a) peut être exécutée par injection d'espèces oxydantes comprenant au moins une des espèces suivantes : oxygène, ozone, protoxyde d'azote.

Les espèces oxydantes peuvent être injectées en continu durant toute la durée du procédé de nettoyage tel que représenté sur la figure 2(a).

Les espèces oxydantes peuvent être injectées selon un flux constant.

Lors de l'injection des espèces oxydantes, une réaction d'oxydation du dépôt métallique 2 se produit en leur surface libre.

Le procédé d'élimination comprend également une étape b) d'injection d'espèces chimiques adaptées pour volatiliser le dépôt métallique oxydé, cette étape b) se produisant au moins en partie pendant le processus d'oxydation, mais débutant après le début de l'étape a).

Cependant, les espèces chimiques adaptées pour volatiliser le dépôt métallique oxydé pourraient également réagir avec le dépôt métallique 2, et ainsi passiver la surface exposée dudit dépôt. Cette réaction de passivation du dépôt métallique 2 est une réaction parasite qui limite ou bloque toute réaction d'oxydation dudit dépôt par les espèces oxydantes.

Afin d'éviter cette réaction parasite, les espèces chimiques adaptées pour volatiliser le dépôt métallique oxydé sont injectées, à l'étape b), selon une séquence d'impulsions (tel que représenté à la figure 2(b)), par un système d'injection 4. Bien que les impulsions illustrées sur la figure 2(b) présentent une forme de créneau, d'autres formes d'impulsions peuvent être envisagées, dès lors que deux impulsions successives sont séparées par un intervalle de temps pendant lequel aucune espèce chimique adaptée pour volatiliser le dépôt métallique oxydé n'est injectée.

L'enceinte 1 peut être maintenue à une température comprise entre 20 et 250°C de manière à maintenir sous forme gazeuse les espèces chimiques adaptées pour volatiliser le dépôt métallique. De préférence, la température de l'enceinte 1 est maintenue à une température comprise entre 20 et 150°C, de préférence encore entre 20 et 100°C.

Afin de rendre le procédé d'élimination optimal, les espèces oxydantes sont injectées dès le début du procédé d'élimination afin d'oxyder au moins au niveau de sa surface libre le dépôt métallique 2.

Ainsi, une couche de dépôt métallique oxydé recouvre le dépôt métallique 2.

De manière avantageuse, la première impulsion de l'étape b) est mise en oeuvre après le début de l'étape a).

Il peut être avantageux de connaître la cinétique d'oxydation du dépôt métallique 2.

La cinétique d'oxydation du dépôt métallique 2 dépend de sa nature et des conditions de sa formation, mais également des conditions d'oxydation de l'étape a).

Toutefois, il est à la portée de l'homme du métier de déterminer de manière empirique la cinétique d'oxydation du dépôt métallique 2.

A cet égard, l'homme du métier pourra se référer au document Guangwen Zhou et al., J. Mater. Res., Vol. 20, No. 7 (1684-1694), Jul 2005.

Ainsi, préalablement à la mise en oeuvre du procédé d'élimination du dépôt métallique 2 sur une surface dans l'enceinte 1, il est avantageux de déterminer les cinétiques d'oxydation du dépôt métallique pour différentes conditions d'oxydation, et dresser des abaques pour chacun des types de dépôt métallique 2 susceptibles d'être déposés sur une surface dans l'enceinte 1.

Lesdits abaques peuvent alors être utilisés pour déterminer la quantité du dépôt métallique oxydé pendant une durée prédéterminée et dans des conditions d'oxydation données.

De ce fait, pour la mise en oeuvre du procédé d'élimination du dépôt métallique 2 sur une surface dans l'enceinte 1, l'utilisation desdits abaques permet de déterminer la quantité du dépôt métallique oxydé à l'étape a) pendant une durée donnée.

Pendant toute la durée de la première impulsion de la séquence d'impulsions de l'étape b), la couche de dépôt métallique oxydé est, au moins en partie, volatilisée par les espèces chimiques.

Une fois ladite première impulsion terminée, le processus recommence, les espèces oxydantes se retrouvent majoritaires dans l'enceinte 1, et par conséquent la réaction d'oxydation du dépôt métallique 2 est la réaction dominante, jusqu'au début de la prochaine impulsion de la séquence d'impulsions de l'étape b).

De manière avantageuse, selon l'invention la durée des impulsions de la séquence d'impulsions est ajustée de sorte que la couche du dépôt métallique oxydé ne soit pas intégralement volatilisée. Ainsi, la portion de couche du dépôt métallique oxydé restante forme une barrière à la passivation du dépôt métallique 2 par les espèces chimiques injectées à l'étape b). La réaction de passivation se trouve alors bloquée.

Dit autrement, au cours de l'injection des espèces chimiques pour volatiliser le dépôt métallique oxydé, lors de l'impulsion la plus tardive de deux impulsions successives, les espèces chimiques sont injectées en quantité sous-stoechiométrique par rapport à la quantité du dépôt métallique oxydé entre lesdites deux impulsions successives.

La quantité sous-stoechiométrique susmentionnée est déterminable par la connaissance à la fois de la quantité du dépôt métallique oxydé à l'étape a) entre deux impulsions de la séquence d'impulsions de l'étape b), et du mécanisme de réaction de volatilisation dudit dépôt métallique oxydé avec les espèces chimiques.

Le mode d'injection des espèces chimiques adaptées pour volatiliser le dépôt métallique oxydé présente ainsi de nombreux avantages.

Le premier avantage est de rendre le procédé d'élimination selon l'invention efficace. En effet, la réaction parasite comprenant la passivation du dépôt métallique 2 par les espèces chimiques injectées est ainsi neutralisée. La neutralisation de ladite réaction parasite évite d'avoir à ouvrir l'enceinte 1, et d'avoir recours à un processus de décontamination de cette dernière.

Le second avantage est de pouvoir contrôler la quantité d'espèces chimiques injectées à l'étape b), et permet ainsi de réduire le coût du procédé d'élimination.

Par ailleurs, la séquence d'impulsion de l'étape b) peut être périodique.

En outre, la séquence d'impulsion de l'étape b) peut présenter une période inférieure à 1 seconde (soit une fréquence supérieure à 1 Hz).

A titre d'exemple, l'étape a) est exécutée par injection d'oxygène selon un débit compris entre 100 et 1000 sccm (centimètre cube par minute), de préférence entre 100 et 500 sccm, par exemple 300 sccm.

Les espèces chimiques adaptées pour volatiliser le dépôt métallique oxydé peuvent comprendre de l'hexafluoroacetylacetone (hfacH).

Les espèces hfacH sont injectées à l'étape b) selon une séquence d'impulsions présentant une période de 1 seconde, la durée de chaque impulsion étant de 100 ms. Pendant chaque impulsion de la période d'impulsions, les espèces hfacH sont injectées selon un débit compris entre 100 et 500 sccm, par exemple 200 sccm.

La température dans la chambre de dépôt est maintenue à 50 °C

Ainsi, lorsque le dépôt métallique 2 comprend du cuivre (Cu), les réactions d'oxydation de l'étape a) sont les suivantes :

2*Cu* + *O*₂ → 2*CuO*

4*Cu* + *O*₂ → 2*Cu*₂*O*

Lors d'une impulsion de la séquence d'impulsions, l'oxydation de l'étape a) devient minoritaire, et le dépôt métallique oxydé est volatilisé par les espèces hfacH injectées à l'étape b) selon les réactions suivantes :

2*H* + 2hfacH + *CuO* → *Cu*(hfac*H*)₂ + *H*₂*O*

2*H* + 2hfacH + *Cu*₂*O* → *Cu* + *Cu*(hfac*H*)₂ + *H*₂*O*

La suite de la description présente deux applications dans lesquelles le procédé d'élimination est mis en oeuvre. Les caractéristiques décrites ci-dessus en référence au procédé d'élimination peuvent être transposées à ces deux applications.

Dans une première application de l'invention, le procédé d'élimination est mis en oeuvre pour le nettoyage de résidus métalliques 2 disposés sur les parois intérieures d'une chambre de dépôt 1.

Dans ce premier mode de réalisation, l'enceinte mentionnée dans le procédé d'élimination est une chambre de dépôt 1. La chambre de dépôt 1 peut être une chambre de dépôt chimique en phase vapeur (CVD), dépôt physique en phase vapeur (PVD), dépôt physique en phase vapeur assistée par plasma (PECVD), dépôt par couches atomiques (ALD).

Lors du dépôt d'un film sur un substrat disposé sur le support 5 de la chambre de dépôt 1, des dépôts indésirables du matériau compris dans le film sont observés sur des surfaces dans la chambre de dépôt 1, à savoir sur les parois intérieures de la chambre de dépôt 1, ainsi que sur le support 5.

Au fil des dépôts mis en oeuvre dans la chambre 1, lesdits dépôts indésirables de matériaux s'accumulent sur ces surfaces, et sont une source de contamination importante des films formés sur les substrats.

Les films formés dans ces chambres de dépôt peuvent être des films métalliques, et les dépôts de matériaux compris dans lesdits films observés sur les parois intérieures de la chambre de dépôt 1 sont appelés résidus métalliques 2.

L'invention consiste alors à nettoyer ces résidus métalliques 2 disposés sur les parois intérieures de la chambre de dépôt 1. De manière analogue au procédé d'élimination d'un dépôt métallique décrit ci-dessus, le procédé conforme au premier mode de réalisation comprend les étapes suivantes :
a) une étape d'oxydation des résidus métalliques 2 ;
b) une étape d'injection d'espèces chimiques adaptées pour volatiliser les résidus métalliques oxydés.

L'étape b) est mise en oeuvre pendant au moins une partie de l'étape a). A l'étape b), les espèces chimiques sont injectées selon une séquence d'impulsions.

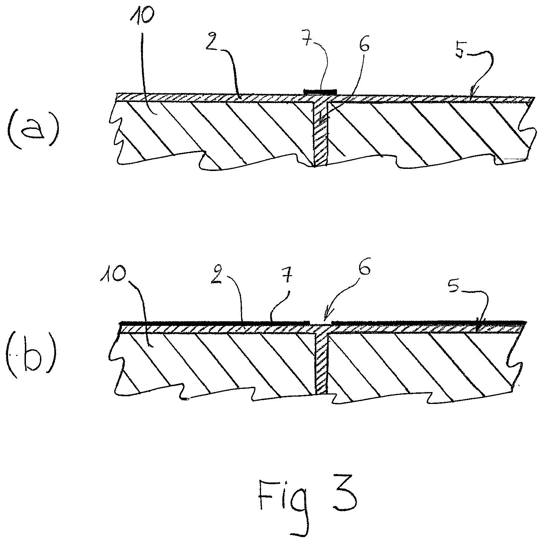

Les figures 3(a) et 3(b) montrent deux variantes d'une deuxième application de l'invention, dans laquelle le procédé d'élimination d'un dépôt métallique 2 est mis en oeuvre pour la fabrication d'un circuit imprimé 10, afin de graver un dépôt métallique 2 déposé sur un substrat du circuit imprimé.

Dans l'exemple des figures 3(a) et 3(b), le dépôt métallique 2 est utilisé pour remplir un via 6. Les vias 6 sont des trous métallisés qui permettent la connexion électrique entre une ou plusieurs couches d'un substrat de circuit imprimé.

Aux figures 3(a) et 3(b), un seul via 6 est représenté, étant entendu que le circuit imprimé 10 peut comporter plusieurs vias 6.

Le procédé de l'invention peut également être mis en oeuvre d'une manière plus générale pour graver un dépôt métallique déposé sur la surface d'un substrat. Par exemple, le procédé permet de réaliser un plot sur un substrat de circuit imprimé, à partir d'une couche de dépôt métallique.

Le substrat du circuit imprimé 10 représenté aux figures 3(a) et 3(b) comporte une face 5. Un via 6 traverse au moins partiellement le circuit imprimé 10 pour connecter électriquement la face 5 à une autre couche du circuit imprimé 10, non représentée.

Le dépôt métallique 2 est réalisé en excès. Il remplit le via 6 afin de le métalliser de manière satisfaisante, et recouvre également la face 5 du circuit imprimé 10. Le procédé de l'invention permet de graver cet excès de dépôt métallique 2, de sorte que le dépôt métallique persiste uniquement à l'intérieur du via 6 et dépasse éventuellement à l'extérieur, le long de son axe longitudinal. Autour du via 6 et sur la surface 5, le dépôt métallique 2 est retiré grâce au procédé de l'invention.

Le circuit imprimé 10 est disposé dans une enceinte non représentée, par exemple une chambre de dépôt similaire à la chambre de dépôt 1 décrite en référence à la première application du procédé de l'invention, pour le nettoyage de résidus métalliques.

La figure 3(a) montre une première variante du deuxième mode de réalisation. Préalablement à la mise en oeuvre du procédé, un masque 7 est apposé sur chaque région du dépôt métallique 2 à conserver, au niveau de la surface 5 du circuit imprimé 10. Le masque 7 est centré sur l'axe longitudinal du via 6.

Puis, les étapes a) et b) du procédé sont mises en oeuvre conformément à l'invention, pour éliminer l'excès du dépôt métallique 2 dans chaque région de la surface 5 non recouverte par le masque 7.

Lors de l'étape a), le dépôt métallique 2 est oxydé uniquement autour du via 6, puisque le masque 7 protège le via 6. Au terme de l'étape a), la partie du dépôt métallique 2 située dans le via 6 et le long de son axe longitudinal n'est pas oxydée.

Lors de l'étape b), des espèces chimiques adaptées pour volatiliser le dépôt métallique 2 oxydé sont injectées dans l'enceinte 1. L'étape b) est mise en oeuvre pendant au moins une partie de l'étape a). A l'étape b), les espèces chimiques sont injectées selon une séquence d'impulsions.

Les espèces chimiques adaptées pour volatiliser le dépôt métallique 2 oxydé agissent uniquement autour du via 6, le masque 7 protégeant le dépôt métallique 2 au niveau du via 6.

Les étapes a) et b) sont répétées autant de fois que nécessaire pour retirer progressivement l'épaisseur de la partie excédante du dépôt métallique 2.

La figure 3(b) montre une deuxième variante du deuxième mode de réalisation. Préalablement à la mise en oeuvre du procédé, un masque 7 est apposé sur chaque région du dépôt métallique 2 à graver, au niveau de la surface 5 du circuit imprimé 10 et autour du via 6.

Puis, des espèces chimiques adaptées pour passiver le dépôt métallique 2 dans chaque région non recouverte par le masque sont injectées dans l'enceinte 1. La passivation peut être effectuée en injectant, en excès par rapport à la quantité du dépôt métallique, les espèces oxydantes et/ou les espèces chimiques adaptées pour volatiliser le dépôt métallique 2 oxydé, de préférence en quantité sur-stoechiométrique par rapport à la quantité du dépôt métallique 2.

Ainsi, le dépôt métallique 2 est passivé uniquement au niveau des zones non masquées, c'est-à-dire au niveau du via 6.

Le masque 7 est ensuite retiré.

Puis, les étapes a) et b) du procédé sont mises en oeuvre conformément à l'invention, pour éliminer l'excès du dépôt métallique 2 sur la surface 5, dans chaque région préalablement recouverte par le masque.

Lors de l'étape a), le dépôt métallique 2 est oxydé uniquement autour du via 6, puisqu'au niveau du via 6, le dépôt métallique est passivé et remplit ainsi une fonction de masque. Au terme de l'étape a), le dépôt métallique n'est pas oxydé au niveau du via 6.

Lors de l'étape b), des espèces chimiques adaptées pour volatiliser le dépôt métallique 2 oxydé sont injectées dans l'enceinte 1. L'étape b) est mise en oeuvre pendant au moins une partie de l'étape a). A l'étape b), les espèces chimiques sont injectées selon une séquence d'impulsions.

Les espèces chimiques adaptées pour volatiliser le dépôt métallique 2 oxydé agissent uniquement autour du via 6, la passivation protégeant le dépôt métallique 2 au niveau du via 6 à la manière d'un masque.

Les étapes a) et b) sont répétées autant de fois que nécessaire pour retirer l'excès du dépôt métallique 2.

Par rapport au procédé conventionnel de gravure d'un dépôt métallique par polissage mécano-chimique, l'invention présente les avantages suivants :
- alors que le polissage mécano-chimique requiert le dépôt d'une couche métallique épaisse (typiquement supérieure ou égale à 1,5 µm) pour assurer l'uniformité du polissage ultérieur, la gravure mise en oeuvre dans l'invention permet de déposer uniquement l'épaisseur de métal suffisante au remplissage du via, soit quelques centaines de nanomètres déposés en excès. L'invention permet donc de diminuer d'environ un facteur 10 la quantité de métal à déposer.
- en s'affranchissant du polissage mécano-chimique qui entraîne une contamination importante du substrat, l'invention évite également les étapes de nettoyage consécutives à ce polissage,
- enfin, la formation d'une couche d'arrêt entre le substrat et le dépôt métallique n'est plus nécessaire.

Ainsi, grâce à l'invention, la gravure d'un dépôt métallique déposé sur une surface est simplifiée et moins coûteuse.

Bien entendu l'invention n'est pas limitée aux modes de mise en oeuvre décrits. On peut y apporter des variantes de réalisation sans sortir du cadre de l'invention.

Ainsi, le procédé d'élimination d'un dépôt métallique selon l'invention permet de limiter les réactions parasites susceptibles de bloquer ledit procédé.

### REFERENCES

G. Zhou et al.: Initial oxidation kinetics of Cu(100), (110), and (111) thin films investigated by in situ UHV TEM, J. Mater. Res., Vol. 20, No. 7, Jul 2005

## Revendications

1. Procédé d'élimination d'un dépôt métallique (2) disposé sur une surface (5) dans une enceinte (1), ledit procédé comprenant les étapes suivantes :
a) une étape d'oxydation du dépôt métallique (2) ;
b) une étape d'injection d'espèces chimiques adaptées pour volatiliser le dépôt métallique oxydé, ladite étape b) étant mise en oeuvre pendant au moins une partie de l'étape a) ;
ledit procédé d'élimination **étant caractérisé en ce que,** à l'étape b), les espèces chimiques sont injectées selon une séquence d'impulsions, et lors de l'impulsion la plus tardive de deux impulsions successives, les espèces chimiques sont injectées en quantité sous-stoechiométrique par rapport à la quantité du dépôt métallique oxydé entre lesdites deux impulsions successives de sorte que le dépôt métallique oxydé ne soit pas intégralement volatilisé.

2. Procédé selon la revendication 1, dans lequel la séquence d'impulsions des espèces chimiques injectées est périodique.

3. Procédé selon la revendication 2, dans lequel la période de la séquence d'impulsions est inférieure à 1 seconde.

4. Procédé selon l'une des revendications 1 à 3, dans lequel l'enceinte (1) est maintenue à une température comprise entre 20 et 250°C, de préférence entre 20 et 150°C.

5. Procédé selon l'une des revendications 1 à 4, dans lequel l'étape a) est exécutée par injection d'une espèce oxydante comprenant au moins une des espèces suivantes : oxygène, ozone, protoxyde d'azote.

6. Procédé selon l'une des revendications 1 à 5, dans lequel les espèces chimiques injectées à l'étape b) comprennent de l'hexafluoroacetylacetone.

7. Procédé selon l'une des revendications 1 à 6, dans lequel le dépôt métallique (2) comprend au moins un des éléments suivants : Cuivre, Titane, Tantale, Ruthénium, Zinc, Zirconium, Vanadium, Argent, Or, Chrome.

8. Procédé selon l'une des revendications 1 à 7, dans lequel la première impulsion de l'étape b) est mise en oeuvre après le début de l'étape a).

9. Procédé selon l'une des revendications 1 à 8, **caractérisé en ce qu'**il est mis en oeuvre pour le nettoyage de résidus métalliques (2) disposés sur les parois intérieures d'une chambre de dépôt (1).

10. Procédé selon l'une des revendications 1 à 8, **caractérisé en ce qu'**il est mis en oeuvre pour graver un dépôt métallique (2) déposé en excès sur une surface (5).

11. Procédé selon la revendication 10, **caractérisé en ce que** préalablement aux étapes a) et b), un masque (7) est apposé de manière localisée sur le dépôt métallique (2).

12. Procédé selon la revendication 11, **caractérisé en ce qu'**il comprend les étapes suivantes :
- le masque (7) est apposé sur chaque région du dépôt métallique (2) à conserver ;
- les étapes a) et b) sont mises en oeuvre pour éliminer l'excès du dépôt métallique (2) dans chaque région non recouverte par le masque.

13. Procédé selon la revendication 11, **caractérisé en ce qu'**il comprend les étapes suivantes :
- le masque (7) est apposé sur chaque région du dépôt métallique (2) à graver ;
- des espèces chimiques adaptées pour passiver le dépôt métallique (2) dans chaque région non recouverte par le masque sont injectées ;
- le masque (7) est retiré ;
- les étapes a) et b) sont mises en oeuvre pour éliminer l'excès du dépôt métallique (2) dans chaque région préalablement recouverte par le masque.

14. Procédé selon l'une des revendications 10 à 13, **caractérisé en ce que** les étapes a) et b) sont répétées autant de fois que nécessaire pour éliminer l'excès du dépôt métallique (2).

## Patentansprüche

1. Verfahren zur Abtragung eines auf einer Oberfläche (5) in einer Kammer (1) aufgetragenen Metallüberzugs (2), wobei das Verfahren folgende Schritte umfasst:
a) einen Schritt zur Oxidation des Metallüberzugs (2);
b) einen Schritt zur Einspritzung von chemischen Spezies, die zur Verflüchtigung des oxidierten Metallüberzugs geeignet sind, wobei Schritt b) während mindestens eines Teils von Schritt a) durchgeführt wird;
wobei das Abtragungsverfahren **dadurch gekennzeichnet ist, dass** in Schritt b) die chemischen Spezies gemäß einer Abfolge von Impulsen eingespritzt werden und dass bei dem spätesten Impuls von zwei aufeinander folgenden Impulsen die chemischen Spezies in unterstöchiometrischer Menge im Vergleich zur Menge des oxidierten Metallüberzugs zwischen den zwei aufeinander folgenden Impulsen derart eingespritzt werden, dass der oxidierte Metallüberzug nicht vollständig verflüchtigt wird.

2. Verfahren nach Anspruch 1, wobei die Abfolge von Impulsen der eingespritzten, chemischen Spezies periodisch ist.

3. Verfahren nach Anspruch 2, wobei die Periode der Abfolge von Impulsen kürzer als 1 Sekunde ist.

4. Verfahren nach einem der Ansprüche 1 bis 3, wobei die Kammer (1) bei einer Temperatur zwischen 20 °C und 250 °C, bevorzugt zwischen 20 °C und 150 °C gehalten wird.

5. Verfahren nach einem der Ansprüche 1 bis 4, wobei Schritt a) durch Einspritzung einer oxidierenden Spezies ausgeführt wird, welche mindestens eine der folgenden Spezies umfasst: Sauerstoff, Ozon, Distickstoffmonoxid.

6. Verfahren nach einem der Ansprüche 1 bis 5, wobei die in Schritt b) eingespritzten chemischen Spezies Hexafluoracetylaceton umfassen.

7. Verfahren nach einem der Ansprüche 1 bis 6, wobei der Metallüberzug (2) mindestens eins der folgenden Elemente umfasst: Kupfer, Titan, Tantal, Ruthenium, Zink, Zirkonium, Vanadium, Silber, Gold, Chrom.

8. Verfahren nach einem der Ansprüche 1 bis 7, wobei der erste Impuls von Schritt b) nach dem Beginn von Schritt a) durchgeführt wird.

9. Verfahren nach einem der Ansprüche 1 bis 8, **dadurch gekennzeichnet, dass** es für die Bereinigung von auf den Innenwänden einer Ablagerungskammer (1) aufgetragenen Metallrückständen (2) ausgeführt wird.

10. Verfahren nach einem der Ansprüche 1 bis 8, **dadurch gekennzeichnet, dass** es zur Ätzung eines auf einer Oberfläche (5) überschüssig aufgetragenen Metallüberzugs (2) ausgeführt wird.

11. Verfahren nach Anspruch 10, **dadurch gekennzeichnet, dass** vor den Schritten a) und b) eine Maske (7) örtlich eingeschränkt auf den Metallüberzug (2) aufgelegt wird.

12. Verfahren nach Anspruch 11, **dadurch gekennzeichnet, dass** es folgende Schritte umfasst:
- die Maske (7) wird auf jeden zu erhaltenden Bereich des Metallüberzugs (2) aufgelegt;
- die Schritte a) und b) werden durchgeführt, um den überschüssigen Metallüberzug (2) in jedem durch die Maske nicht abgeschirmten Bereich abzutragen.

13. Verfahren nach Anspruch 11, **dadurch gekennzeichnet, dass** es folgende Schritte umfasst:
- die Maske (7) wird auf jeden zu ätzenden Bereich des Metallüberzugs (2) aufgelegt;
- chemische Spezies werden eingespritzt, welche zur Passivierung des Metallüberzugs (2) in jedem durch die Maske nicht abgeschirmten Bereich geeignet sind;
- die Maske (7) wird entfernt;
- die Schritte a) und b) werden durchgeführt, um den überschüssigen Metallüberzug (2) in jedem vorab durch die Maske abgeschirmten Bereich abzutragen.

14. Verfahren nach einem der Ansprüche 10 bis 13, **dadurch gekennzeichnet, dass** die Schritte a) und b) so oft wie nötig wiederholt werden, um den überschüssigen Metallüberzug (2) abzutragen.

## Claims

1. Method for removing a metallic deposit (2) disposed on a surface (5) in an enclosure (1), said method comprising the following steps:
a) a step of oxidizing the metallic deposit (2);
b) a step of injecting chemical species capable of volatilizing the oxidized metallic deposit, said step b) being implemented during at least a part of step a);
said removal method **being characterized in that** in step b), the chemical species are injected according to a sequence of pulses, during the latest pulse of two successive pulses, the chemical species are injected in a sub-stoichiometric quantity with respect to the quantity of the oxidized metallic deposit between said two successive pulses so that the oxidized metallic deposit is not entirely volatilized.

2. Method according to claim 1, in which the sequence of pulses of the injected chemical species is periodic.

3. Method according to claim 2, in which the period of the sequence of pulses is less than 1 second.

4. Method according to one of claims 1 to 3, in which the enclosure (1) is maintained at a temperature comprised between 20 and 250°C, preferably between 20 and 150°C.

5. Method according to one of claims 1 to 4, in which step a) is carried out by injecting an oxidizing species comprising at least one of the following species: oxygen, ozone, nitrous oxide.

6. Method according to one of claims 1 to 5, in which the chemical species injected in step b) comprise hexafluoroacetylacetone.

7. Method according to one of claims 1 to 6, in which the metallic deposit (2) comprises at least one of the following elements: Copper, Titanium, Tantalum, Ruthenium, Zinc, Zirconium, Vanadium, Silver, Gold, Chromium.

8. Method according to one of claims 1 to 7, in which the first pulse of step b) is implemented after the start of step a).

9. Method according to one of claims 1 to 8, **characterized in that** it is implemented for cleaning off metallic residues (2) disposed on the inner walls of a deposition chamber (1).

10. Method according to one of claims 1 to 8, **characterized in that** it is implemented for etching a metallic deposit (2) deposited in excess on a surface (5).

11. Method according to claim 10, **characterized in that** before steps a) and b), a mask (7) is affixed in a localized manner onto the metallic deposit (2).

12. Method according to claim 11, **characterized in that** it comprises the following steps:
- the mask (7) is affixed over each region of the metallic deposit (2) to be retained;
- steps a) and b) are implemented in order to remove the excess metallic deposit (2) in each region not covered by the mask.

13. Method according to claim 11, **characterized in that** it comprises the following steps:
- the mask (7) is affixed over each region of the metallic deposit (2) to be etched;
- chemical species adapted to passivate the metallic deposit (2) in each region not covered by the mask are injected;
- the mask (7) is removed;
- steps a) and b) are implemented in order to remove the excess metallic deposit (2) in each region covered beforehand by the mask.

14. Method according to one of claims 10 to 13, **characterized in that** steps a) and b) are repeated as many times as necessary in order to remove the excess metallic deposit (2).
